# FASCICULE DE BREVET EUROPEEN

(11) **EP 2 660 594 B1**
(45) Date de publication et mention de la délivrance du brevet: **12.11.2014**
(21) Numéro de dépôt: 13165959.1
(22) Date de dépôt: 30.04.2013
(51) Int. Cl.: G01N 33/14, G01N 9/26, G01N 9/36

(54) **Appareil électronique de suivi de vinification**
Elektronisches Gerät zur Überwachung der Weinbereitung
Electronic apparatus for tracking vinification

(30) Priorité: 03.05.2012 FR 1201289
(43) Date de publication de la demande: 06.11.2013
(73) Titulaire: Systel Electronique, 71390 Cersot (FR)
(72) Inventeur: Julien, Laurent, 21000 DIJON (FR); Garneret, Bernard, 21550 LADOIX SERRIGNY (FR); Malsert, Jean-Emmanuel, 21220 CHAMBOEUF (FR)
(74) Mandataire: Oudin, Stéphane

(56) Documents cités:
- EP-A1- 0 271 380
- DE-A1- 10 064 010
- FR-A1- 2 639 710

## Description

### Domaine technique

La présente invention concerne le domaine général des instruments de mesure et a pour objet un appareil électronique de suivi de vinification.

### Technique antérieure

La vinification, c'est-à-dire la transformation par fermentation d'un moût de raisin en vin, et notamment la transformation des sucres contenus dans le moût en alcool, est un processus complexe et sensible à de nombreux facteurs (température, humidité, etc).

Afin de surveiller l'évolution de ce processus, les vignerons déterminent le taux de sucre et d'alcool du mélange en fermentation à intervalles de temps réguliers, ce qui permet d'en déduire la vitesse de la réaction de fermentation notamment.

Plusieurs méthodes sont appliquées à cette fin.

Une première méthode consiste à utiliser un densimètre (également dénommé hydromètre ou mustimètre), instrument permettant de mesurer la masse volumique d'un liquide (ici, le moût) en utilisant le principe d'Archimède. Il consiste en un cylindre creux, lesté et gradué, qui s'enfonce plus ou moins dans le liquide à mesurer selon sa densité. En utilisant des tables de correspondance, on en déduit la teneur en sucres et en alcool du moût en cours de vinification : le moût en vinification est en effet assimilable à un mélange d'eau, de sucres et d'alcool, renfermant également des gaz en suspension et des particules solides.

Ce système de mesure, très peu onéreux (quelques euros à quelques dizaines d'euros) et très facile à mettre en oeuvre dans l'environnement direct d'une cuverie vinicole, présente toutefois plusieurs inconvénients. Tout d'abord, la mesure est manuelle et n'est pas automatisable : on verse un peu de moût dans un cylindre mesureur et on y plonge le mustimètre qui doit flotter librement ; on lit ensuite l'échelle graduée au ras du liquide ; puis le cas échéant on reporte la valeur mesurée dans un système de traitement informatisé. Ensuite, cette méthode est très sensible à la présence de gaz dans le liquide. Enfin, le prélèvement est réalisé habituellement par le biais d'un robinet situé sur la face avant de la cuve : l'échantillon prélevé n'est donc pas représentatif de l'ensemble de la cuve.

La demande de brevet français FR 2 639 710 A1 divulgue un dispositif électronique de mesure de la densité de liquides, permettant en particulier le suivi de la variation de cette densité au cours des procédures de fermentation, comportant, dans une variante, un capteur de pression et un détecteur de niveau indiquant la hauteur de liquide au-dessus du capteur de pression, installé dans le récipient pour le liquide. La demande de brevet allemande DE 100 64 010 A1 divulgue également un dispositif de mesure de la densité d'un liquide basé sur le principe d'Archimède, comportant une colonne de mesure munie d'un raccordement pour la mesure de la pression.

Une autre méthode connue pour le suivi de la vinification est de mesurer le taux de CO2, qui augmente au cours de la fermentation. Des systèmes électroniques réalisant cette mesure sont proposés sur le marché. Ils ont toutefois un coût plutôt élevé. De plus, cette méthode a pour contrainte de nécessiter une étanchéité soignée des cuves de fermentation, afin d'éviter toute contamination pouvant fausser la mesure.

La présente invention vise donc à obvier un certain nombre des inconvénients des dispositifs connus et à proposer un appareil électronique qui permette de réaliser des mesures de suivi de vinification, éventuellement de façon automatique, sans risque de contaminer le moût, et de les exporter pour traitement informatisé, qui soit précis, fiable et robuste dans un environnement agro-industriel et dont le prix de revient le rende accessible.

### Résumé de l'invention

La présente invention concerne un appareil pour le suivi de la vinification comprenant un dispositif de mesure au moins, une unité de traitement du signal produit par le(s) dispositif(s) de mesure et un châssis supportant lesdites unités remarquable en ce qu'il comporte un dispositif de mesure de densité d'un liquide comprenant une colonne de mesure munie d'un premier capteur de pression placé en pied de colonne et d'un moyen de détermination de la hauteur du liquide dans la colonne de mesure et en ce que le sommet de la colonne est pourvu d'un dispositif de valve anticontamination contenant une solution aqueuse de SO2 permettant de neutraliser, lors de l'opération de vidange de ladite colonne, l'oxygène contenu dans l'air extérieur aspiré afin d'éviter de contaminer le moût réinjecté dans le milieu duquel il a été prélevé.

Le dispositif de valve anticontamination comporte avantageusement un réservoir muni d'un raccord étanche à l'extrémité supérieure de la colonne, un tube de communication inséré dans ledit raccord, le sommet dudit réservoir étant fermé par un couvercle comportant un clapet anti-retour et un tube plongeur d'aspiration et étant rempli par la solution aqueuse de SO2 de sorte que l'extrémité supérieure du tube de communication soit émergée tandis que l'extrémité inférieure du tube soit immergée, tout en évitant des débordements de ladite solution aqueuse de SO2 dans le tube de communication.

De préférence, le moyen de détermination de la hauteur de liquide dans la colonne de mesure est un second capteur de pression, disposé le long de ladite colonne, vers son sommet.

On comprend bien que le recours à au moins un capteur de pression permet d'obtenir un appareil de mesure peu sensible à la température externe et faiblement sensible à la présence de gaz ou d'autres particules dans le liquide.

### Brève description des figures

D'autres avantages et caractéristiques ressortiront mieux de la description qui va suivre, donnée à titre d'exemple non limitatif, d'une variante d'exécution particulièrement préférée de la présente invention, en référence aux dessins annexés sur lesquels :
- la figure 1 est une vue de face de l'appareil;
- la figure 2 est une vue en coupe longitudinale sagittale selon l'axe II-II' de la figure 1 ;
- la figure 3 est une vue de détail partielle de la figure 2 ;
- la figure 4 est une vue en perspective et en éclaté du détail représenté à la figure 3.

### Description détaillée de l'invention

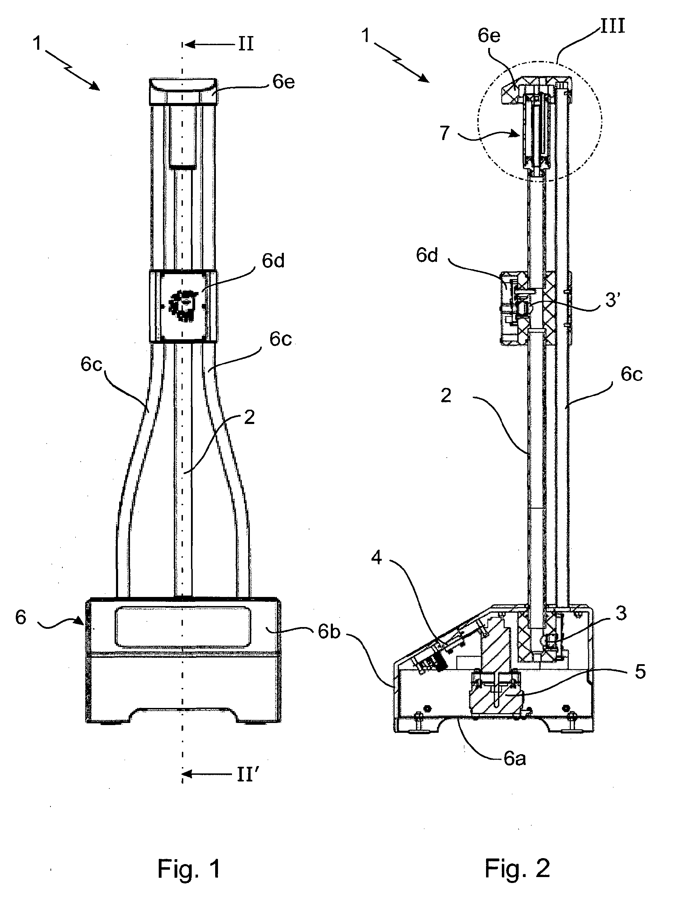

En référence aux figures 1 et 2, l'appareil 1 comporte au moins un dispositif de mesure de densité, ou densimètre, comprenant une colonne 2 de mesure munie d'un premier capteur de pression 3 placé en pied de colonne et d'un moyen de détermination de la hauteur du liquide dans la colonne 2 de mesure.

Selon la variante d'exécution préférée représentée sur les figures, ce moyen de détermination de la hauteur du liquide dans la colonne 2 de mesure est un second capteur de pression 3', disposé le long de ladite colonne 2, vers son sommet. Ce choix s'avère en effet très fiable et précis et aisé à mettre en oeuvre.

Les capteurs de pression 3 ,3' sont disposés de sorte à être en contact direct avec le liquide dont la densité doit être déterminée. Ainsi, une découpe est prévue dans la colonne 2 de sorte que la membrane du second capteur 3' soit à fleur de la paroi interne de ladite colonne 2.

Dans le cadre de la présente invention, d'autres moyens de détermination de la hauteur du liquide dans la colonne 2 peuvent être mis en oeuvre. Ainsi par exemple, un capteur optique avec un signal lumineux réfléchi peut être utilisé. Un système à flottation couplé à un interrupteur peut aussi être envisagé.

La colonne 2 de mesure fait au moins 40 cm de haut, de préférence entre 60 et 140 cm.

L'appareil comporte une unité de traitement 4 du signal produit par le(s) dispositif(s) de mesure. Cette unité de traitement 4 est avantageusement constituée par une carte électronique. L'unité de traitement 4 reçoit l'ensemble des signaux produits par les capteurs de l'appareil 1. Elle comporte une interface utilisateur du type écran et clavier, qui peuvent être intégrés à l'appareil 1 ou externe à celui-ci, et peut être connectée à des moyens informatiques externes, via des ports ou des protocoles de communication habituellement utilisés dans ce domaine (par exemple, USB, Bluetooth, WIFI, etc.). L'unité de traitement 4 applique automatiquement des corrections aux données mesurées en fonction de tables prédéfinies (abaques) et/ou de l'étalonnage de l'appareil, en particulier et avantageusement la correction de la densité mesurée en fonction de la température du moût relevée dans la colonne 2 de mesure. Ainsi, l'unité de traitement 4 permet à l'utilisateur d'obtenir directement la valeur de densité corrigée, sans avoir besoin d'appliquer une correction selon des abaques à la valeur affichée par l'appareil 1.

L'appareil 1 comporte en outre un dispositif 5 de pompage et de refoulement dans ladite colonne 2 du liquide dont la densité est mesurée. La pompe de ce dispositif 5 de pompage est de préférence du type péristaltique. Ce dispositif 5 est situé sous la colonne 2. Le premier capteur de pression 3 est placé au niveau du raccordement entre le dispositif 5 de pompage et la colonne 2.

Le tout est supporté par un châssis 6. Selon la variante d'exécution représentée, ce châssis comporte une embase 6a supportant notamment le dispositif 5 de pompage, un capot 6b recouvrant ladite embase 6a protégeant l'unité de traitement 4 et le premier capteur de pression 3. Le châssis 6 comporte également des tubes 6c de support, reliant le capot 6b à un boitier 6d intermédiaire au niveau duquel le deuxième capteur de pression 3' est placé, et une coiffe 6e. Les tubes 6c de support sont avantageusement creux et leur espace interne sert au passage de câbles de connexion.

Selon une caractéristique avantageuse, l'appareil 1 comporte en outre un dispositif de nettoyage de la colonne 2 par rinçage, non représenté.

De préférence, ce dispositif de nettoyage permet de réaliser un bullage, c'est-à-dire l'injection de bulles de gaz dans le liquide de rinçage: on peut ainsi décrocher efficacement les particules du moût qui adhéreraient à la paroi interne de la colonne 2 de mesure.

De préférence, le liquide de rinçage est compatible avec un usage alimentaire et sera de préférence de l'eau, additionnée ou non d'agents nettoyants compatibles avec un usage alimentaire.

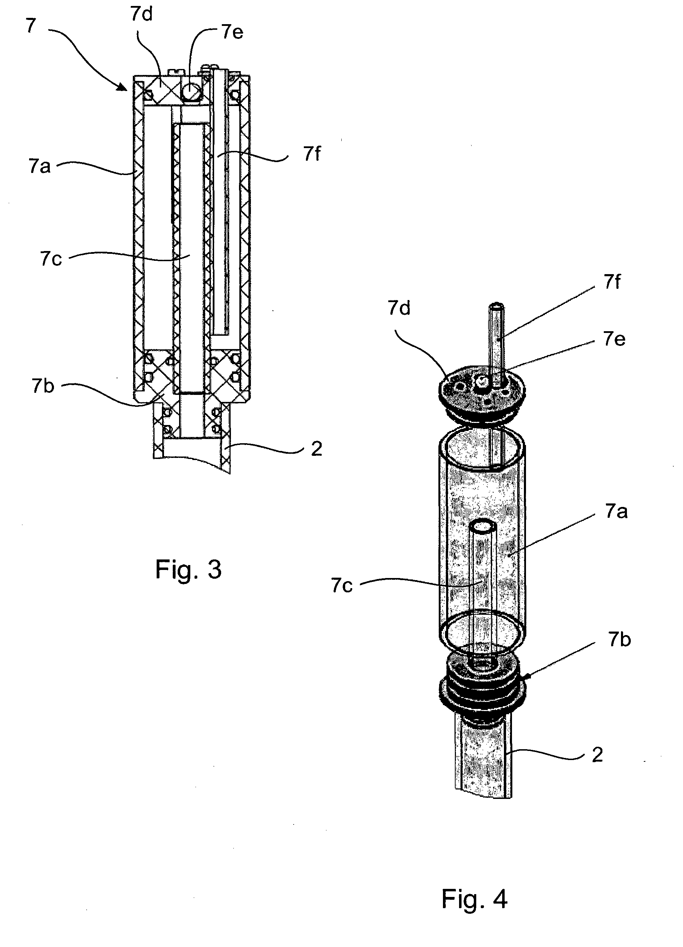

En référence aux figures 3 et 4, le sommet de la colonne 2 est pourvu d'un dispositif de valve 7 anti-contamination. Avantageusement, cette valve 7 comporte un réservoir 7a muni d'un raccord 7b étanche à l'extrémité supérieure de la colonne 2. Dans ce raccord 7b un tube 7c de communication est inséré. Le sommet du réservoir 7a est fermé par un couvercle 7d comportant un clapet 7e anti-retour et un tube 7f plongeur d'aspiration. Le réservoir 7a contient une solution aqueuse de SO2 et est rempli de sorte que l'extrémité supérieure du tube 7c de communication soit émergée tandis que l'extrémité inférieure du tube 7f soit immergée, tout en évitant des débordements de ladite solution aqueuse de SO2 dans le tube 7c de communication.

Lors du remplissage de la colonne 2, l'air qu'elle contient est expulsé au travers du clapet 7e. Pendant l'opération de vidange, l'air extérieur est aspiré par le tube plongeur 7f et traverse la solution aqueuse de SO2, ce qui permet de neutraliser l'oxygène qu'il contient et évite ainsi de contaminer le moût évacué. La mesure peut ainsi être réalisée en circuit fermé, le liquide prélevé étant réinjecté dans le milieu duquel il a été prélevé après la mesure.

Avec le recours au dispositif de nettoyage, l'appareil peut ainsi être connecté à un système de prélèvement multicuves, permettant d'effectuer les mesures pour plusieurs cuves successivement sans déplacement de l'appareil.

De plus, l'appareil 1 peut comporter avantageusement des dispositifs de mesure supplémentaires ou être connecté à d'autres dispositifs de mesure, permettant de suivre d'autres paramètres du moût en cours de vinification. Ainsi, on prévoira des capteurs de température destinés à mesurer la température du liquide dans la colonne 2. Comme exposé précédemment, cela permet avantageusement à l'appareil 1 d'afficher des valeurs de densité corrigées en fonction de la température mesurée. Des capteurs de gaz, notamment CO2 et azote peuvent aussi être prévus en tête de colonne 2.

En outre et avantageusement, l'appareil 1 comporte un dispositif d'automatisation de la prise de mesure. Ce dispositif contrôlera avantageusement le système de prélèvement et l'export des données de mesure déterminée par l'unité de traitement 4 : on peut ainsi mettre en place un programme automatisé de suivi et d'alerte de l'évolution de la fermentation du moût dans une cuve, en se basant sur les valeurs de densité relevées, conjuguées éventuellement à la mesure d'autres paramètres caractérisant la fermentation, tels que la température du moût dans la cuve de fermentation surveillée ou la teneur en CO2.

Selon un mode de réalisation préféré, la colonne 2 est au moins translucide, de préférence transparente : on peut ainsi visualiser la couleur et la turbidité du liquide analysé. Par exemple, la colonne sera réalisée en verre ou en plastique alimentaire. Avantageusement, la portion de la colonne située entre le second capteur 3' et le sommet de ladite colonne 2 est au moins translucide, préférentiellement transparente, et éventuellement graduée : en effet, le remplissage de la colonne est déterminé par le second capteur de pression 3', en fonction d'une valeur de pression prédéterminée et fixe ; comme la densité du liquide varie, la hauteur du liquide va varier en proportion dans la portion supérieure de la colonne 2. La graduation permet alors à un utilisateur extérieur d'avoir une approximation grossière de la densité, juste après le remplissage de la colonne 2 et de déceler une anomalie de la fermentation. De plus, on prévoira avantageusement le long de la paroi de la colonne 2 de mesure un moyen d'éclairage, afin d'améliorer la visualisation du liquide.

Selon une caractéristique de l'invention, les deux capteurs 3,3' sont distants le long de la colonne 2 d'au moins 20 cm, de préférence entre 40 et 100 cm, bornes incluses. Cela assure une fiabilité accrue de la précision de la mesure effectuée.

Selon une caractéristique préférée, les deux capteurs de pression sont appairés. Leurs étalonnages puis respectivement leurs prises de mesures sont donc corrélés. Cela permet avantageusement de n'avoir qu'une seule référence de capteur de pression, dont la dérive de température sera identique.

Afin d'obtenir une précision de la mesure fiable et reproductible, on procédera avantageusement en usine à un étalonnage en pression du densimètre, en faisant suivre aux capteurs de pression une courbe de pression, suivi d'une étalonnage en température à pression constante. Ces données d'étalonnage sont mémorisées de façon permanente dans l'appareil. Enfin, l'appareil est étalonné en mesure de densité avec des liquides étalons dont les densités sont parfaitement connues. Cette dernière opération peut être effectuée par l'utilisateur final.

Enfin, l'appareil 1 sera avantageusement associé dans sa mise en oeuvre à un système de prélèvement comportant une canne plongeant au coeur de la cuve de vinification : la mesure résultante est alors plus homogène et représentative de l'ensemble de la cuve que celle obtenue par prélèvement avec un robinet placé en façade de la cuve.

### Application industrielle

L'appareil électronique selon l'invention est particulièrement destiné à une utilisation dans les exploitations vinicoles en tant que mustimètre afin de déterminer les teneurs en alcool et en sucre du moût au cours de la vinification.

## Revendications

1. Appareil (1) électronique pour le suivi de la vinification comprenant un dispositif de mesure au moins, une unité de traitement (4) du signal produit par le(s) dispositif(s) de mesure et un châssis (6) renfermant lesdites unités, ledit appareil comportant un dispositif de mesure de la densité d'un liquide comprenant une colonne (2) de mesure munie d'un premier capteur de pression (3) placé en pied de colonne et d'un moyen de détermination de la hauteur du liquide dans la colonne (2) de mesure, et le sommet de la colonne (2) étant pourvu d'un dispositif de valve (7) anticontamination contenant une solution aqueuse de S02 permettant de neutraliser, lors de l'opération de vidange de ladite colonne (2), l'oxygène contenu dans l'air extérieur aspiré afin d'éviter de contaminer le moût réinjecté dans le milieu duquel il a été prélevé.

2. Appareil (1) selon la revendication 1 dans lequel le dispositif de valve (7) anticontamination comporte un réservoir (7a) muni d'un raccord (7b) étanche à l'extrémité supérieure de la colonne (2), un tube (7c) de communication inséré dans ledit raccord (7b), le sommet dudit réservoir (7a) étant fermé par un couvercle (7d) comportant un clapet (7e) anti-retour et un tube (7f) plongeur d'aspiration et étant rempli par la solution aqueuse de S02 de sorte que l'extrémité supérieure du tube (7c) de communication soit émergée tandis que l'extrémité inférieure du tube (7f) soit immergée, tout en évitant des débordements de ladite solution aqueuse de S02 dans le tube (7c) de communication.

3. Appareil (1) selon l'une des revendications précédentes dans lequel le moyen de détermination de la hauteur du liquide dans la colonne (2) de mesure est un second capteur de pression (3') disposé le long de ladite colonne (2), vers son sommet.

4. Appareil (1) selon l'une des revendications précédentes comportant en outre un dispositif (5) de pompage et de refoulement dans ladite colonne (2) du liquide dont la densité est mesurée.

5. Appareil (1) selon l'une des revendications précédentes comportant en outre un dispositif de nettoyage de la colonne (2) par rinçage.

6. Appareil (1) selon la revendication précédente comportant un dispositif de nettoyage de la colonne (2) par rinçage avec bullage.

7. Appareil (1) selon l'une des revendications précédentes comportant en outre des capteurs de température destinés à mesurer la température du liquide dans la colonne (2).

8. Appareil (1) selon la revendication précédente
dans lequel l'unité de traitement (4) corrige automatiquement la densité mesurée du liquide dans la colonne (2) en fonction de sa température mesurée.

9. Appareil (1) selon l'une des revendications 3 à 8 dans lequel les deux capteurs de pression (3,3') sont distants le long de la colonne (2) d'au moins 20 cm, de préférence entre 40 et 100 cm, bornes incluses.

10. Appareil (1) selon l'une des revendications 4 à 8 dans lequel le dispositif de pompage (5) est relié à un système de prélèvement multicuve.

## Patentansprüche

1. Elektronisches Gerät (1) zur Überwachung der Weinbereitung, das zumindest eine Messvorrichtung, eine Einheit (4) zur Verarbeitung des Signals, das von der (den) Messvorrichtung(en) erzeugt wird und ein Chassis (6) umfasst, das die besagten Einheiten einschließt, wobei das besagte Gerät eine Vorrichtung zum Messen der Dichte einer Flüssigkeit mit einer Messsäule (2) umfasst, die mit einem ersten Drucksensor (3) versehen ist, der am Boden der Säule platziert ist, und eine Vorrichtung zur Bestimmung des Flüssigkeitspegels in der Messsäule (2), und die Spitze der Säule (2) mit einer Ventilvorrichtung (7) zum Kontaminierungsschutz versehen ist, die eine wässrige S02-Lösung enthält, mit der der beim Entleeren der besagten Säule (2) aus der Außenluft angesaugte Sauerstoff neutralisiert werden kann, um zu vermeiden, den Weinmost zu kontaminieren, wenn dieser wieder in das Milieu eingeführt wird, aus dem er entnommen wurde.

2. Gerät (1) nach Anspruch 1, in dem die Ventilvorrichtung (7) zum Kontaminierungsschutz einen Behälter (7a) mit einem dichten Anschluss (7b) am oberen Ende der Säule (2) enthält, ein Kommunikationsrohr (7c), das in den besagten Anschluss (7b) eingeführt wird, wobei der besagte Behälter (7a) oben durch einen Deckel (7d) verschlossen wird, der ein Rückschlagventil (7e) und ein Saugtauchrohr (7f) enthält, und mit der wässrigen S02-Lösung gefüllt ist, sodass das obere Ende des Kommunikationsrohres (7c) herausragt, während das untere Ende des Rohres (7f) eingetaucht ist, und dabei Überläufe der besagten wässrigen S02-Lösung in das Kommunikationsrohr (7c) vermeidet.

3. Gerät (1) nach einem der vorherigen Ansprüche, in dem die Vorrichtung zur Bestimmung des Flüssigkeitspegels in der Messsäule (2) ein zweiter Drucksensor (3') ist, der entlang der besagten Säule (2) bis zu deren Spitze angeordnet ist.

4. Gerät (1) nach einem der vorherigen Ansprüche, das darüber hinaus eine Vorrichtung (5) zum Pumpen und Umwälzen der Flüssigkeit in die besagte Säule (2) enthält, deren Dichte gemessen wird.

5. Gerät (1) nach einem der vorherigen Ansprüche, das darüber hinaus eine Vorrichtung zum Reinigen der Säule (2) durch Spülen enthält.

6. Gerät (1) nach dem vorherigen Anspruch, das eine Vorrichtung zum Reinigen der Säule (2) durch Spülen mit Blasenbildung enthält.

7. Gerät (1) nach einem der vorherigen Ansprüche, das darüber hinaus Temperaturfühler enthält, die dazu bestimmt sind, die Temperatur der Flüssigkeit in der Säule (2) zu messen.

8. Gerät (1) nach dem vorherigen Anspruch, in dem die Verarbeitungseinheit (4) die gemessene Dichte der Flüssigkeit in der Säule (2) automatisch je nach deren gemessener Temperatur korrigiert.

9. Gerät (1) nach einem der Ansprüche 3 bis 8, in dem die beiden Drucksensoren (3, 3') entlang der Säule (2) mindestens 20 cm, vorzugsweise zwischen 40 und 100 cm inklusive der Klemmen voneinander entfernt sind.

10. Gerät (1) nach einem der Ansprüche 4 bis 8, in dem die Pumpvorrichtung (5) mit einer Mehrbehälter-Entnahmevorrichtung verbunden ist.

## Claims

1. Electronic apparatus (1) for monitoring wine making, comprising at least one measuring device, a unit for processing (4) the signal produced by the measuring device or devices and a frame (6) containing said units, said apparatus comprising a device for measuring the density of a liquid, comprising a measuring column (2) provided with a first pressure sensor (3) placed at the foot of the column and a means for determining the height of the liquid in the measuring column (2), and the top of the column (2) being provided with an anticontamination valve device (7) containing an aqueous solution of SO₂ for neutralising, during the operation of draining said column (2), the oxygen contained in the outside air sucked in in order to avoid contaminating the must reinjected into the medium from which it was taken.

2. Apparatus (1) according to claim 1, wherein the anticontamination valve device (7) comprises a reservoir (7a) provided with a sealed coupling (7b) at the top end of the column (2), a communication tube (7c) inserted in said coupling (7b), the top of said reservoir (7a) being closed by a cover (7d) comprising a non-return valve (7e) and a suction plunger tube (7f), and being filled with the aqueous solution of SO₂ so that the top end of the communication tube (7c) is above the fluid level while the bottom end of the tube (7f) is submerged, while preventing overflows of said aqueous solution of SO₂ in the communication tube (7c).

3. Apparatus (1) according to one of the preceding claims, wherein the means for determining the height of the liquid in the measuring column (2) is a second pressure sensor (3') disposed along said column (2), towards the top thereof.

4. Apparatus (1) according to one of the preceding claims, further comprising a device (5) for pumping and driving, in said column (2), the liquid of which the density is measured.

5. Apparatus (1) according to one of the preceding claims, further comprising a device for cleaning the column (2) by rinsing.

6. Apparatus (1) according to the preceding claim, comprising a device for cleaning the column (2) by rinsing with bubbling.

7. Apparatus (1) according to one of the preceding claims, further comprising temperature sensors intended to measure the temperature of the liquid in the column (2).

8. Apparatus (1) according to the preceding claim, wherein the processing unit (4) automatically corrects the measured density of the liquid in the column (2) according to its measured temperature.

9. Apparatus (1) according to one of claims 3 to 8, wherein the two pressure sensors (3, 3') are distant along the column (2) by at least 20 cm, preferably between 40 and 100 cm, including terminals.

10. Apparatus (1) according to one of claims 4 to 8, wherein the device (5) for pumping is linked to a multi-vat sampling system.
